# EUROPEAN PATENT APPLICATION

(11) **EP 0 998 940 A1**
(43) Date of publication of application: **10.05.2000**
(21) Application number: 98402403.4
(22) Date of filing: 30.09.1998
(51) Int. Cl.: A61K 38/09, A61K 47/40

(54) **Pharmaceutical compositions based on alpha-cyclodextrin for the oral administration of LH-RH analogues**

(71) Applicant: LABORATOIRE THERAMEX, 98000 Monaco (MC)
(72) Inventor: Delansorne, Rémi, 06000 Nice (FR); Bonnet, Paule, 06500 Menton (FR); Paris, Jacques, 06100 Nice (FR)
(74) Representative: Nevant, Marc

(57) **Abstract**

The invention relates to the use of α-cyclodextrin for the preparation of pharmaceutical compositions for the oral administration of LH-RH peptide analogues. The invention also relates to oral pharmaceutical compositions containing LH-RH peptide analogues in combination with α-cyclodextrin.

## Description

The present invention relates to the pharmaceutical field. More specifically, the invention relates to the use of α-cyclodextrin or derivatives thereof for the preparation of pharmaceutical compositions for the oral administration of LH-RH (luteinizing hormone - releasing hormone) peptide analogues. The invention also relates to oral pharmaceutical compositions containing LH-RH peptide analogues in combination with α-cyclodextrin.

Natural and modified cyclodextrins (CDs) are well known ingredients used in a large variety of pharmaceutical preparations taking advantage of one or several of their properties relating to drug solubilization and stabilization (Loftsson and Brewster, 1996, *J. Pharm. Sci*, **85**(10): 1017-1025) or to overall improvement of *in vivo* drug delivery (Rajewski and Stella, 1996, *J. Pharm. Sci.,* **85**(11): 1142-1169). CDs are cyclic oligosaccharides containing at least 6 α-D-(+)-glucopyranose units attached by α(1-4) glucoside bonds (Nash, *Handbook of Pharmaceutical Excipients,* ed. by Wade and Weller, 1994, American Pharmaceutical Association, Washington, and The Pharmaceutical Press, London, pp 145-148); the three most common CDs are α-, β- and γ-CD which consist of 6, 7 and 8 sugar units, respectively. Numerous derivatives of each type of CD can be obtained by random or controlled modifications of one, several or all free hydroxyl groups of the sugar moities.

LH-RH is a neurohormone produced by hypothalamic neurons and secreted in the pituitary portal vasculature to stimulate the release of luteinizing hormone (LH) and follicle stimulating hormone (FSH) by the pituitary gland. In turn, LH and FSH regulate the endocrine and germinal functions of the ovary in the female and of the testis in the male. LH-RH is a peptide of the following structure: pGlu-His-Trp-Ser-Tyr-Gly-Leu-Arg-Pro-Gly-NH₂. Numerous normal or reduced-size, linear or cyclic peptide analogues of LH-RH incorporating natural, unusual or chemically-modified amino-acids have been synthesized over the years to yield potent agonist or antagonistic properties (Karten and Rivier, 1986, *Endocr. Rev.*, **7**(1): 44-66; Dutta, 1988, *Drugs of the Future,* **13**(8): 761-787; Kutscher et al., 1997, *Angew. Chem. Int. Ed. Engl*, **36**: 2148-2161). Due to their total or partial peptide structure, however, all these analogues show poor oral bioavailability and bioactivity.

To date, only non-oral administration of LH-RH peptide analogues, has been reported. For example, Matsubara et al. (1996, J. Pharm. Sci., **84**(11) : 1295-1300) describe a nasal formulation of buserelin, based on dimethyl-β-CD, with improved bioavailability.

There is therefore a need, for the patients' comfort, to provide formulations which enable the oral administration of LH-RH peptide analogues.

It has now surprisingly been found that α-CD or its derivatives, enhance the biological activity of LH-RH peptide analogues when orally administered.

Thus, according to one of its feature, the invention relates to the use of α-cyclodextrin or derivatives thereof for the preparation of pharmaceutical compositions for the oral administration of LH-RH peptide analogues.

Examples of LH-RH peptide analogues which can be used within the scope of the invention include those described in International patent applications WO 98/21 229 and PCT/EP98/02802 (filed on May 6, 1998), as well as standard agonists and antagonists of LH-RH, such as for example buserelin, nafarelin, leuprorelin, goserelin, histrelin, triptorelin, deslorelin, lutrelin, avorelin, cetrorelix, antide, ganirelix, azaline B, antarelix, detirelix, ramorelix or teverelix.

Preferably, these peptide analogues have the formula (SEQ ID N°:1):
A1-A2-A3-A4-A5-A6-A7-A8-Pro - Z (A)
in which :
- A1 is pGlu; D-pGlu ; Sar ; AcSar ; Pro or a derivative thereof such as AcPro, ForPro, OH-Pro, Ac-OH-Pro, dehydro-Pro or Ac-dehydro-Pro ; Ser ; D-Ser ; Ac-D-Ser; Thr ; D-Thr ; Ac-D-Thr ; or an aromatic D-amino acid which may be acylated, such as D-Phe, D-HPhe, D-Tyr, D-HTyr, D-Trp, D-2MeTrp, D-Nal, D-1Nal, D-diphenyl-Ala, D-Bal, D-Pal, D-4Pal or D-Qal, where D-Phe, D-HPhe, D-Tyr, D-HTyr, and D-Trp may be substituted by one or more halogens, (C₁-C₄)alkyl, (C₁-C₄)alkoxy, nitro or trifluoromethyl groups;
- A2 is a direct bond ; His ; or an aromatic D-amino acid such as D-Phe, D-HPhe, D-Tyr, D-HTyr, D-Trp, D-2MeTrp, D-Nal, D-1Nal, D-diphenyl-Ala, D-Bal, D-Pal, D-4Pal or D-Qal, where D-Phe, D-HPhe, D-Tyr, D-HTyr and D-Trp may be substituted by one or more halogens, (C₁-C₄)alkyl, (C₁-C₄)alkoxy, nitro or trifluoromethyl groups;
- A3 is an aromatic L- or D-amino acid such as Phe, HPhe, Tyr, HTyr, Trp, 2MeTrp, Nal, 1Nal, diphenyl-Ala, Bal, Pal, 4Pal or Qal, where Phe, HPhe, Tyr, HTyr and Trp may be substituted by one or more halogens, (C₁-C₄)alkyl, (C₁-C₄)alkoxy, nitro or trifluoromethyl groups;
- A4 is Ala, Ser, D-Ser, MeSer, Ser(OBu^{t}), Ser(OBzl) or Thr;
- A5 is an aromatic L-amino acid such as Phe, HPhe, Tyr, HTyr, Trp, 2MeTrp, Nal, 1Nal, diphenyl-Ala, Bal, Pal, 4Pal or Qal, where Phe, HPhe, Tyr, HTyr and Trp may be substituted by one or more halogens, (C₁-C₄)alkyl, (C₁-C₄)alkoxy, nitro or trifluoromethyl groups ; or a basic L- or D-amino acid such as Arg, HArg, Orn, Lys, HLys, Cit, HCit, APhe or ACha, where Arg and HArg may be N-substituted by a (C₁-C₆)alkyl or a (C₃-C₆)cycloalkyl group on one or both nitrogen atoms, and where Orn, Lys, HLys, APhe and ACha may be N-substituted by one or two (C₁-C₆)alkyl or (C₃-C₆)cycloalkyl groups, or by an aminotriazolyl or a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;
- A6 is Gly ; (S)-spirolactam-Pro ; D-Pro ; D-Ser ; D-Thr ; D-Cys ; D-Met ; D-Pen ; D-(S-Me)Pen ; D-(S-Et)Pen ; D-Ser(OBu^{t}) ; D-Asp(OBu^{t}) ; D-Glu(OBu^{t}) ; D-Thr(OBu^{t}) ; D-Cys(OBu^{t}) ; D-Ser(OR₁) where R₁ is a sugar moiety; an aza-amino acid such as azaGly or azaAla ; D-His which may be substituted on the imidazole ring by a (C₁-C₆)alkyl, a (C₂-C₇)acyl or a benzyl group ; an aliphatic D-amino acid with a (C₁-C₈)alkyl or a (C₃-C₆)cycloalkyl side chain such as D-Ala, D-Abu, D-Aib, D-3Aib, D-Val, D-Nva, D-Leu, D-Ile, D-Tle, D-Nle, D-Hol, D-Npg, D-CPa, D-Cpa, D-Cba or D-Cha ; an aromatic D-amino acid such as D-Phe, D-HPhe, D-Tyr, D-HTyr, D-Trp, D-2MeTrp, D-Nal, D-1Nal, D-diphenyl-Ala, D-anthryl-Ala, D-phenanthryl-Ala, D-benzhydryl-Ala, D-fluorenyl-Ala, D-Bal, D-Pal, D-4Pal or D-Qal, where D-Phe, D-HPhe, D-Tyr, D-HTyr and D-Trp may be substituted by one or more halogens, (C₁-C₄)alkyl, (C₁-C₄)alkoxy, nitro or trifluoromethyl groups ; D-cyclohexadienyl-Gly ; D-perhydronaphthyl-Ala ; D-perhydrodiphenyl-Ala ; or a basic L- or D-amino acid such as Arg, HArg, Orn, Lys, HLys, Cit, HCit, APhe or ACha, where Arg and HArg may be N-substituted by a (C₁-C₆)alkyl or a (C₃-C₆)cycloalkyl group on one or both nitrogen atoms, and where Orn, Lys, HLys, APhe and ACha may be N-substituted by one or two (C₁-C₆)alkyl or (C₃-C₆)cycloalkyl groups, or by an aminotriazolyl or a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;
- A7 is a linear, branched or cyclic aliphatic L-amino acid of 3 to 20 carbon atoms such as Ala, Abu, Aib, 3Aib, Val, Nva, Leu, Ile, Tle, Nle, Hol, Npg, CPa, Cpa, Cba, Cha or Ada, which may be N-alpha-substituted by a (C₁-C₄)alkyl group optionally substituted by one or several fluorine atoms ;
- A8 is a basic L- or D-amino acid such as Arg, HArg, Cm, Lys, HLys, Cit, HCit, APhe or ACha, where Arg or HArg may be N-substituted by a (C₁-C₆)alkyl or a (C₃-C₆)cycloalkyl group on one or both nitrogen atoms, and where Orn, Lys, HLys, APhe or ACha may be N-substituted by one or two (C₁-C₆)alkyl or (C₃-C₆)cycloalkyl groups, or by an aminotriazolyl or a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;
- Z is GlyNH₂ ; D-AlaNH₂ ; azaGlyNH₂ ; or a group -NHR₂ where R₂ is a (C₁-C₄)alkyl which may be substituted by an hydroxy or one or several fluorine atoms ; a (C₃-C₆)cycloalkyl ; or a heterocyclic radical selected from morpholinyl, pyrrolidinyl and piperidyl;
as well as their pharmaceutically acceptable salts.

In the present description the term "(C₁-C₄)alkyl" denotes methyl, ethyl, n-propyl, i-propyl, n-butyl, i-butyl, s-butyl and t-butyl groups.

The term "(C₁-C₆)alkyl" denotes methyl, ethyl, n-propyl, -propyl, n-butyl, i-butyl, s-butyl, t-butyl, n-pentyl, i-pentyl, s-pentyl, t-pentyl and hexyl groups.

The term "(C₁-C₈)alkyl" denotes methyl, ethyl, n-propyl, 1-propyl, n-butyl, i-butyl, s-butyl, t-butyl, n-pentyl, i-pentyl, s-pentyl, t-pentyl, hexyl, heptyl and octyl groups ;

The term "(C₁-C₄)alkoxy" denotes a group -OR where R is a (C₁-C₄)alkyl.

The term "(C₂-C₇)acyl" denotes a group -COR where R is a (C₁-C₆)alkyl.

The term "(C₃-C₆)cycloalkyl" denotes cyclopropyl, cyclobutyl, cyclopentyl and cyclohexyl groups.

The term "sugar moiety" denotes D- or L-pentoses or hexoses and their amino-derivatives.

The term "LH-RH analogues" denotes peptides in which at least one amino acid has been modified in the sequence of LH-RH.

The term "(S)spirolactam-Pro" denotes the residue of the formula :

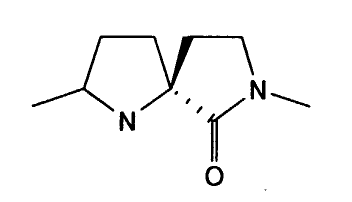

Peptidomimetic analogues of LH-RH defined by the absence of at least one peptide amide bond, as exemplified in the latest review by Kutscher et al. (1997, *Angew. Chem. Int. Ed. Engl.*, **36**: 2148-2161), are not considered within the scope of the present invention.

In the present description and in the claims, the following abbreviations are used :

| | |
|---|---|
| Abu : 2-aminobutyric acid | Ac : acetyl |
| ACha : aminocyclohexylalanine | Aib : 2-aminoisobutyric acid |
| 3Aib : 3-aminoisobutyric acid | Ala : alanine |
| AlaNH₂: alaninamide | APhe : p-aminophenylalanine |
| Arg : arginine | Asp : aspartic acid |
| azeAla : aza-alanine | azaGly : aza-glycine |
| azaGlyNH₂ : azaglycinamide | Bal : benzothienylalanine |
| Boc : *tert*-butoxycarbonyl | Cba : cyclobutylalanine |
| Cha : cyclohexylalanine | Cit : citrulline |
| CPa : cyclopropylalanine | Cpa : cylopentylalanine |
| Fmoc : fluorenylmethoxycarbonyl | For : formyl |
| Glu : glutamic acid | Gly : glycine |
| GlyNH₂ : glycinamide | HArg : homoarginine |
| HCit : homocitrulline | His : histidine |
| HLys : homolysine | Hol : homoleucine |
| Ile : isoleucine | IprLys: Nε-isopropyllysine |
| Leu : leucine | Lys : lysine |
| MeSer : N-methylserine | Met : methionine |
| Nal : 3-(2-naphtyl)alanine | 1Nal : 3-(1-naphtyl)alanine |
| NEt : N-ethylamide | NicLys : Nε-nicotinoyllysine |
| Nle : norleucine | Npg : neopentylglycine |
| Nva : norvaline | OBu^{t} : *tert*-butoxy |
| OBzl : benzyl ester | Orn : ornithine |
| Pal : 3-(3-pyridyl)alanine | pClPhe : 3-(4-chlorophenyl)alanine |
| Pen : penicillamine | pGlu : pyroglutamic acid |
| Phe : phenylalanine | Pro : proline |
| Qal : 3-(3-quinolyl)alanine | Sar : sarcosine |
| Ser : serine | (S-Me)Pen : S-methyl-penicillamine |
| (S-Et)Pen : S-ethyl-penicillamine | Thr : threonine |
| Tle : tert-leucine | Trp : tryptophan |
| Tyr : tyrosine | Val : valine |
| Ada : adamantylalanine | HPhe : homophenylalanine |
| MeNpg : N-methylneopentylglycine | 4Pal : 3-(4-pyridyl)alanine |
| HTyr : homotyrosine | 2MeTrp : 2-methyltryptophan |
| Bzl : benzyl | SPL : (S)spirolactam-Pro |

A preferred group of peptide analogues (A) comprise the peptides of the formula (SEQ ID N° : 2) :
A1-His-A3-A4-A5-A6-A7-A8-Pro-Z (I)
in which:
- A1 is pGlu, Sar or AcSar;
- A3 is an aromatic L-amino acid such as Phe, HPhe, Tyr, HTyr, Trp, 2MeTrp, Nal, 1Nal, diphenyl-Ala, Bal, Pal, 4Pal or Qal, where Phe, HPhe, Tyr, HTyr and Trp may be substituted by one or more halogens, (C₁-C₄)alkyl, (C₁-C₄)alkoxy, nitro or trifluoromethyl groups;
- A4 is Ala, Ser, D-Ser, MeSer, Ser(OBu^{t}), Ser(OBzl) or Thr;
- A5 is an aromatic L-amino acid such as Phe, HPhe, Tyr, HTyr, Trp, 2MeTrp, Nal, 1Nal, diphenyl-Ala, Bal, Pal, 4Pal or Qal, where Phe, HPhe, Tyr, HTyr and Trp may be substituted by one or more halogens, (C₁-C₄)alkyl, (C₁-C₄)alkoxy, nitro or trifluoromethyl groups;
- A6 is Gly ; D-Pro ; (S)-spirotactam-Pro ; D-Ser ; D-Thr ; D-Cys ; D-Met ; D-Pen ; D-(S-Me)Pen ; D-(S-Et)Pen ; D-Ser(OBu^{t}) ; D-Asp(OBu^{t}) ; D-Glu(OBu^{t}) ; D-Thr(OBu^{t}) ; D-Cys(OBu^{t}) ; D-Ser(OR₁) where R₁ is a sugar moiety ; an aza-amino acid such as azaGly or azaAla ; D-His which may be substituted on the imidazole ring by a (C₁-C₆)alkyl or a benzyl group ; an aliphatic D-amino acid with a (C₁-C₈)alkyl or a (C₃-C₆)cycloalkyl side chain such as D-Ala, D-Abu, D-Aib, D-3Aib, D-Val, D-Nva, D-Leu, D-Ile, D-Tle, D-Nle, D-Hol, D-Npg, D-CPa, D-Cpa, D-Cba or D-Cha ; an aromatic D-amino acid such as D-Phe, D-HPhe, D-Tyr, D-HTyr, D-Trp, D-2MeTrp, D-Nal, D-1Nal, D-diphenyl-Ala, D-anthryl-Ala, D-phenanthryl-Ala, D-benzhydryl-Ala, D-fluorenyl-Ala, D-Bal, D-Pal, D-4Pal or D-Qal, where D-Phe, DHPhe, D-Tyr, D-HTyr and D-Trp may be substituted by one or more halogens, (C₁-C₄)alkyl, (C₁-C₄)alkoxy, nitro or trifluoromethyl groups; D-cyclohexadienyl-Gly ; D-perhydronaphtyl-Ala ; D-perhydrodiphenyl-Ala ; or a basic D-amino acid such as D-Arg, D-HArg, D-Orn, D-Lys, D-HLys, D-Cit, D-HCit, D-APhe optionally substituted by an aminotriazolyl group or D-ACha, where D-Arg and D-HArg may be be N-substituted by a (C₁-C₆)alkyl or (C₃-C₆)cycloalkyl groups, or by a Fmoc or Boc group;
- A7 is a linear, branched or cyclic aliphatic L-amino acid of 3 to 20 carbon atoms such as Ala, Abu, Aib, 3Aib, Val, Nva, Leu, Ile, Tle, Nle, Hol, Npg, CPa, Cpa, Cba, Cha or Ada, which may be N-alpha-substituted by a (C₁-C₄)alkyl group optionally substituted by one or several fluorine atoms;
- A8 is a basic L-amino acid such as Arg, HArg, Orn, Lys, HLys, Cit, HCit, APhe optionally substituted by an aminotriazolyl group, or ACha;
- Z is GlyNH₂ ; azaGlyNH₂ ; or a group -NHR₂ where R₂ is a (C₁-C₄)alkyl which may be substituted by an hydroxy or one or several fluorine atoms ; a (C₃-C₆)cycloalkyl ; or a heterocyclic radical selected from morpholinyl, pyrrolidinyl and piperidyl;
as well as their pharmaceutically acceptable salts.

Among the peptide analogues of formula (I), those of the formula (SEQ ID N° : 3) :
pGlu-His-A3-Ser-A5-A6-A7-Arg-Pro-Z (II)
in which:
- A3 and A5 are aromatic L-amino acids as defined for (I) ;
- A6 is as defined for (I) ;
- A7 is Leu, Tle, Nle, Hol, Npg, Cha or Ada, which may be N-alpha-substituted by a methyl or ethyl group optionally substituted by one or several fluorine atoms;
- Z is as defined for (I) ;
as well as their pharmaceutically acceptable salts,
are preferred.

Especially preferred are the peptide analogues of the formula (SEQ ID N° : 4) :
pGlu-His-A3-Ser-A5-A6-A7-Arg-Pro-Z (III)
in which:
- A3 and A5 are each independently Phe, Tyr, Trp, 2MeTrp, HPhe, HTyr, Nal, 1Nal, Bal, Pal, 4Pal, or pClPhe ;
- A6 is (S)-spirolactam-Pro ; Gly; D-Pro ; D-Ser(OBu^{t}) ; D-Asp(OBu^{t}) ; D-Glu(OBu^{t}) ; D-Thr(OBu^{t}) ; D-Cys(OBu^{t}); D-His or D-His(Bzl) ; D-Ala, D-Leu, D-Tle, D-Nle, D-Hol, D-Npg or D-Cha ; D-Phe, D-HPhe, D-Tyr, D-HTyr, D-Trp, D-2MeTrp, D-Nal, D-1Nal, D-Bal, D-Pal, D-4Pal, or D-pClPhe; D-cyclohexadienyl-Gly ; D-perhydronaphtyl-Ala ; D-perhydrodiphenyl-Ala ; or D-APhe optionally substituted by an aminotriazolyl group ;
- A7 is Leu, Npg or Cha, which may be N-alpha-substituted by a methyl group;
- Z is GlyNH₂ ; azaGlyNH₂ or -NC₂H₅ ;

Examples of the salts with pharmaceutically acceptable acids are those with mineral acids, such as for example the hydrochloride, hydrobromide, sulfate, phosphate, borate, hydrogensulfate, dihydrogenphosphate or nitrate, and those with organic acids, such as for example the acetate, oxalate, tartrate, succinate, maleate, fumarate, gluconate, citrate, pamoate, malate, ascorbate, benzoate, p-toluenesulfonate or naphtalenesulfonate.

Examples of the salts with pharmaceutically acceptable bases are those with alkali or alkaline earth metals such as sodium, potassium, calcium or magnesium, and those with organic bases such as amines, trometamol, N-methylglutamine, and the like.

The peptides used in the present invention can be prepared by the well-known techniques of peptide chemistry such as for example peptide synthesis in solution or solid phase peptide synthesis. In general, these techniques involve the stepwise addition of one or more amino acids -which may be suitably protected- to a forming peptide chain. Reference can for example be made to *Synthetic Peptides: a user's guide*, ed. by G.A. Grant, 1992, UWBC Biotechnical Resource Series, Washington University Press, Saint-Louis, USA.

Each molecule of α-CD bears 6 primary hydroxyl groups and 12 secondary hydroxyl groups, respectively correspondind to the 6-OH and to the 2- and 3-OH groups of each of the 6 glucopyranose units. Another general aspect of the present invention concerns α-CD and its derivatives which are defined as the result of chemical or biochemical modifications involving a precise or average number between 1 and 18 hydroxyl groups of the α-CD molecule, in a random or regioselective fashion, with one or several different types of reactions such as oxidation, reduction, alkylation, hydroxyalkylation, esterification with organic or mineral acids, intramolecular dehydration, tosylation followed by reductive amination or halogen substitution, sugar branching or further polymerization, and their different possible combinations and mixtures. Examples of α-CD derivatives include α-CD modified with one or more groups selected from methyl, carboxymethyl, ethyl, butyl, octyl, hydroxyethyl, hydroxypropyl, hydroxybutyl, acetyl, propionyl, butyryl, succinyl, benzoyl, palmityl, sulfonyl, toluenesulfonyl, amino, aminopropyl, glucosyl, maltosyl, dimaltosyl, carboxymethyl ether, sulfobutylether, and phosphate ester.

As mentioned above, α-CD or its derivatives enhance the biological activity of LH-RH peptide analogues in oral pharmaceutical compositions.

Thus, according to another feature, the invention relates to oral pharmaceutical compositions which comprise as the active principle a LH-RH peptide analogue in the form of a combination with α-CD or a derivative thereof.

Preferably, the peptide analogue used in the pharmaceutical composition of the invention is a peptide of the general formula (I).

The peptides according to the general formula (I) exert an agonist activity upon the LH-RH receptors *in vivo*, resulting in the stimulation of LH secretion by the pituitary, which, in males, stimulates the secretion of testosterone by the testis.

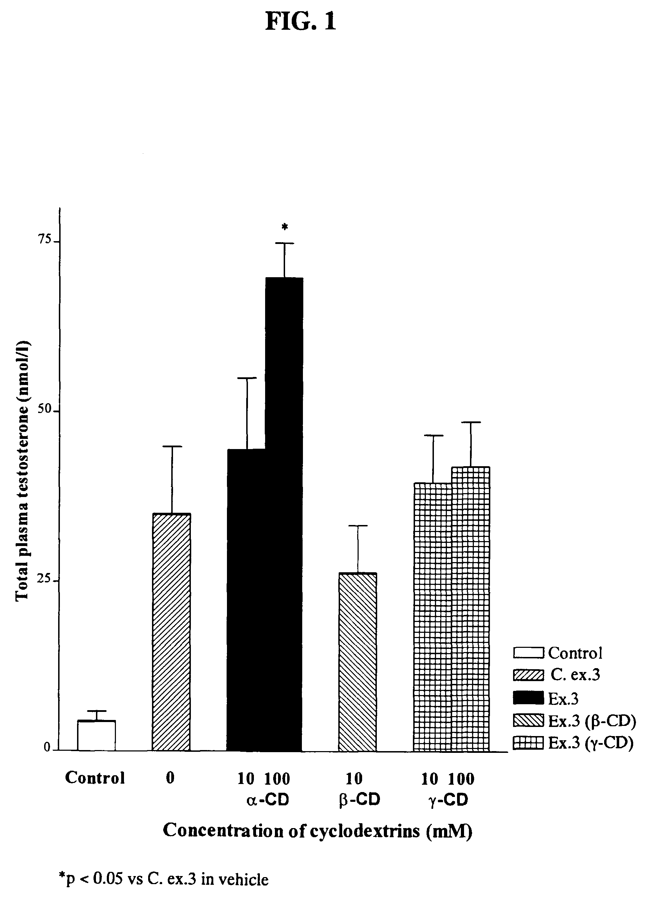

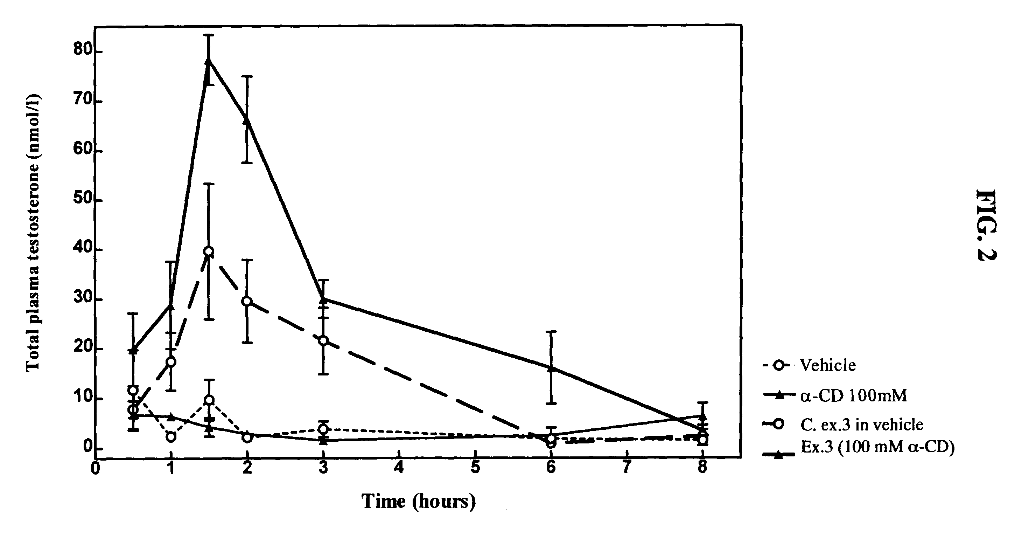

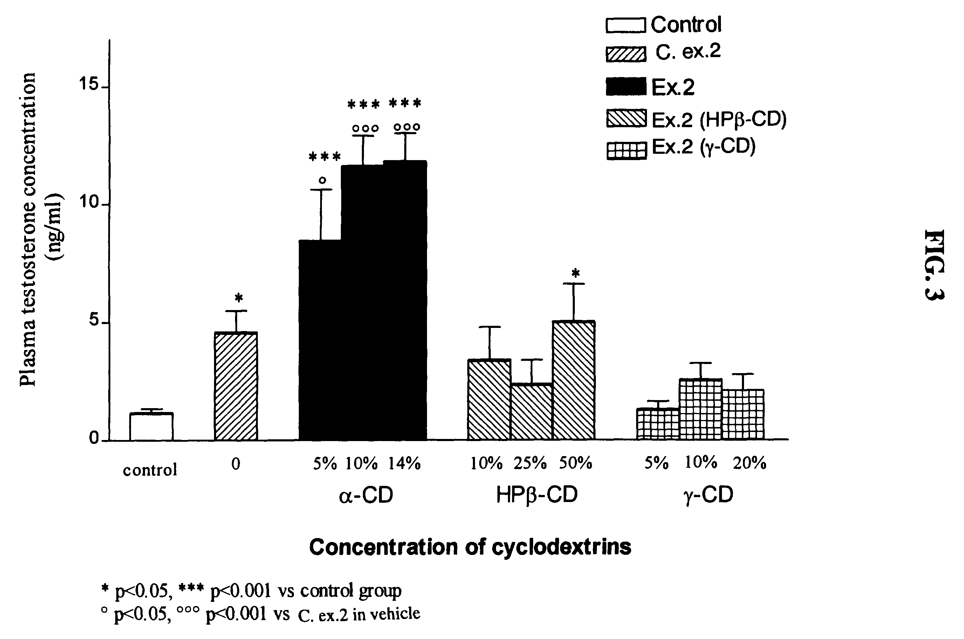

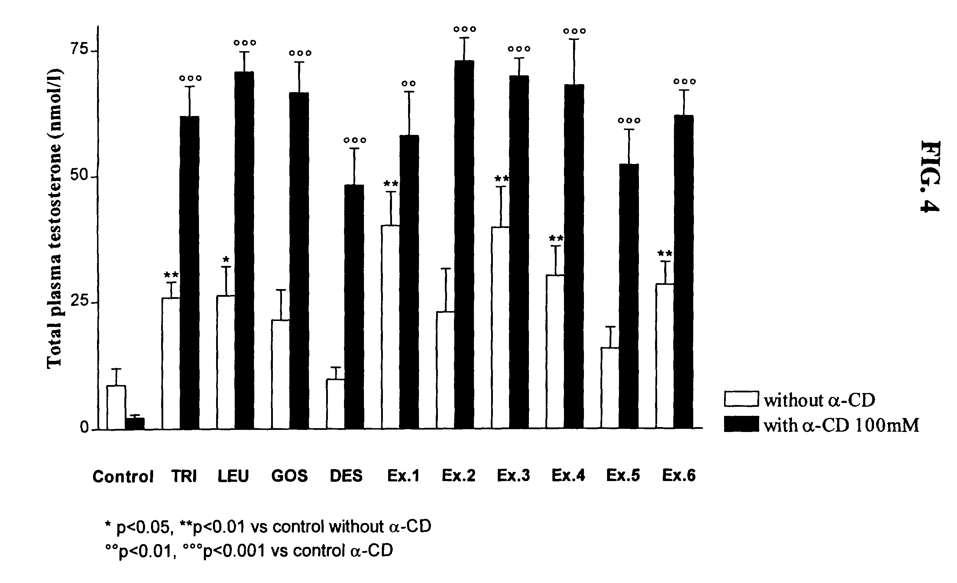

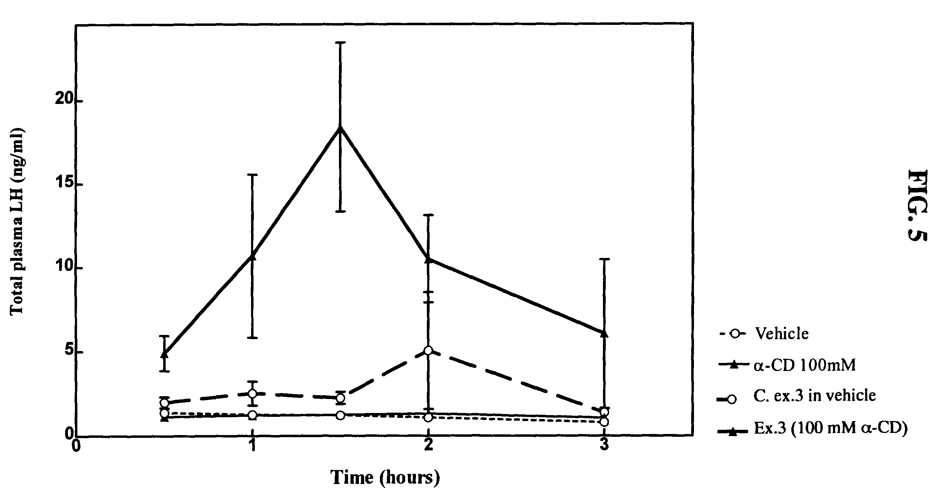

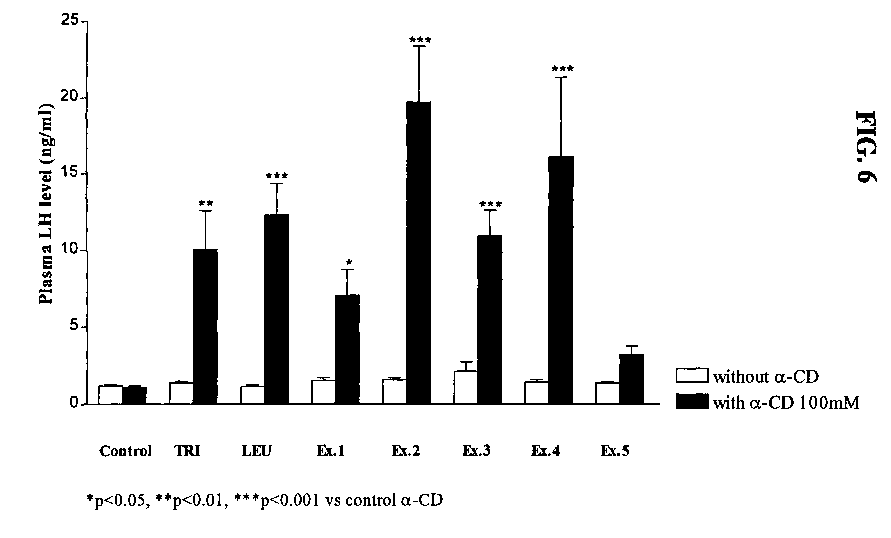

Adult male Sprague-Dawley rats were administered an oral formulation comprising leuprorelin (LEU, Bachem), triptorelin (TRI, Bachem), deslorelin (DES, Saxon Biochemicals), goserelin (GOS, Saxon Biochemicals) or the other following example analogues: example 1 ([(S)spirolactam(Pro⁶, Npg⁷), desGly¹⁰-ProNEt⁹]LH-RH), example 2 ([D-Ala⁶, Npg⁷, desGly¹⁰-ProNEt⁹]LH-RH), example 3 ([Npg⁷]leuprorelin), example 4 ([D-Phe⁶, Npg⁷, desGly¹⁰-ProNEt⁹]LH-RH), example 5 ([Npg⁷]triptorelin) and example 6 ([D-Ala⁸, desGly¹⁰-ProNet⁹]LH-RH, Bachem), in combination with α-CD (Sigma or Wacker Chemie). As a comparison, the same agonists have been orally administered in a standard aqueous vehicle not comprising α-CD (comparative examples). For screening purposes, blood samples were drawn 2 hours after oral administration of a common dose of 2 nmoles/rat of LH-RH peptide agonists in an aqueous solution containing 10 or 100 mM of α-CD (Tables 1 and 2; Figures 1, 4 and 6). For kinetic purposes, the effects of 2 nmoles/rat of example 3 with or without 100 mM of α-CD were tested between 0.5 and 8 hours on plasma LH and testosterone levels (Figures 2 and 5). The influence of increasing concentrations of α-CD (5%, 10% or 14%) was tested with example 2 at the dose of 5 µg/kg 2 hours after oral administration (Figure 3). Total plasma testosterone (Diagnostic System Laboratories) and LH (Amersham Pharmacia Biotech) determinations were performed by radioimmunoassay. In screening 2-hour experiments, each group comprised between 6 and 8 rats; each time point of the kinetic study was studied on four animals.

**Table 1**

| stimulation of testosterone secretion | | | |
|---|---|---|---|
| Compound without α-CD | total plasma testosterone (nmol/l) (m ± sem) | Compound with α-CD | total plasma testosterone (nmol/l) (m ± sem) |
| Control | 8.6 ± 3.32 | Control (α-CD) | 3.8 ± 0.66 |
| Triptorelin | 25.8 ± 3.14 | Triptorelin (α-CD) | 61.9 ± 6.01 |
| Leuprorelin | 26.3 ± 5.77 | Leuprorelin (α-CD) | 70.7 ± 4.06 |
| Goserelin | 21.4 ± 5.99 | Goserelin (α-CD) | 66.5 ± 6.19 |
| Deslorelin | 9.7 ± 2.41 | Deslorelin (α-CD) | 48.2 ± 7.29 |
| C. ex 1 | 40.1 ± 6.78 | Example 1 | 58.0 ± 8.75 |
| C. ex 2 | 23.0 ± 8.54 | Example 2 | 72.8 ± 4.64 |
| C. ex 3 | 39.7 ± 8.11 | Example 3 | 69.7 ± 3.6 |
| C. ex 4 | 30.1 ± 5.86 | Example 4 | 67.9 ± 9.11 |
| C. ex 5 | 15.8 ± 4.24 | Example 5 | 52.1 ± 6.99 |
| C. ex 6 | 28.3 ± 4.56 | Example 6 | 61.8 ± 5.10 |

As can be seen from the above results as well as from Figures 1-4, oral formulations with α-CD significantly enhance the stimulation of testosterone secretion induced by LH-RH analogues. Especially, deslorelin alone was inactive at this threshold dose of 2 nmoles/rat, but showed a marked potency when formulated with α-CD. The crucial role played by α-CD is demonstrated by the concentration-dependance of its effect : combined with 10 mM α-CD (0.972%), the oral activity of example 3 was not significantly improved (Figure 1); at 5% (51.4 mM), α-CD did enhance the stimulation of testosterone secretion induced by example 2 by oral administration, although not to the maximal level achieved with 10% (103 mM) as well as 14% (144 mM) (Figure 3).

It is also worth noting (see figures 1 and 3) that β-CD, hydroxypropyl-β-CD (HP-β-CD) and γ-CD have no potentiating effect on the LH-RH analogue-induced stimulation of testosterone secretion.

**Table 2**

| stimulation of LH secretion | | | |
|---|---|---|---|
| Compound without α-CD | total plasma LH (ng/ml) (m ± sem) | Compound with α-CD | total plasma LH (ng/ml) (m ± sem) |
| Control | 1.2 ± 0.11 | Control (α-CD) | 1.1 ± 0.10 |
| Triptorelin | 1.4 ± 0.10 | Triptorelin (α-CD) | 10.1 ± 2.54 |
| Leuprorelin | 1.2 ± 0.14 | Leuprorelin (α-CD) | 12.3 ± 2.03 |
| C. ex 1 | 1.5 ± 0.19 | Example 1 | 7.1 ± 1.68 |
| C. ex 2 | 1.6 ± 0.14 | Example 2 | 19.7 ± 3.70 |
| C. ex 3 | 2.2 ± 0.58 | Example 3 | 10.9 ± 1.66 |
| C. ex 4 | 1.4 ± 0.17 | Example 4 | 16.1 ± 5.22 |
| C. ex 5 | 1.4 ± 0.10 | Example 5 | 3.2 ± 0.56 |

As can be seen from the above results as well as from Figures 5-6, the potentiating effect of α-CD in oral formulations containing LH-RH analogues on LH release is even more pronounced than on testosterone secretion : all tested LH-RH analogues were inactive when administered alone at the same dose of 2 nmoles/rat, whereas, depending on the analogue, they induced a 3- to 16-fold increase above control levels when administered in combination with α-CD.

The oral formulations of the invention can be prepared by methods well known to those skilled in the art, generally as follows : a known amount of a drug is added to an aqueous cyclodextrin solution in sufficient concentration; the drug-cyclodextrin interaction can take place in solution or suspension within minutes or after stirring for up to 1 week at the desired temperature with or without sonication, depending on the nature of the drug and of the cyclodextrin, and on their respective concentrations. Then, the resulting drug-cyclodextrin combination or complex can be further obtained in a dry form by filtration, centrifugation, evaporation or sublimation.

By way of illustration, the example combinations of LH-RH analogues with α-CD hereafter describe one basic method for the preparation of the formulations according to the invention in solution, notwithstanding their further processing to any appropriate dry form that will take advantage of the same potentiating properties.

Such formulations may further include, one or several other pharmaceutically appropriate excipients for oral administration such as lactose, fructose, glucose, sucrose, compressible sugar, saccharin, povidone, crospovidone, magnesium stearate, kaolin, bentonite, colloidal silica, mannitol, sorbitol, starch and its derivatives, microcrystalline or powdered cellulose, methylcellulose, carboxymethylcellulose, ethylcellulose or other chemically modified celluloses, other cyclodextrins, maltodextrin, dextrates, dextrin, dextrose, alginates, pectins, pectates, sorbitan esters, polysorbate 80, chitosan, guar or xanthan gums, mono-, di- or tri-ethanolamine, oleic acid or ethyl oleate, stearic acid, water, liquid glucose, propylene glycol, lactic acid, malic acid, ethanol, isopropyl myristate or palmitate, glycerin, glyceryl monooleate, glyceryl monostearate, glyceryl palmitostearate, lecithin, medium or short chain triglycerides, various oils from corn, cottonseed, olive, peanut, sesame or soybean, and the like. Those formulations may generally be administered by mouth (or naso-gastric tubing) in various aqueous or non-aqueous solutions or suspensions such as true solutions, syrups, elixirs, mucilages, jellies, gels, milks, magmas, macro-, micro-or nano-emulsions, or in various solid forms such as compressed, coated, buccal, sublingual, effervescent or molded tablets, hard or soft capsules, pills, troches or cachets. Enteric coatings of usual solid oral dosage forms or of soft capsules containing liquid formulations, and sustained, delayed or programmed gastric, enteric or colonic release forms or devices are preferred means to deliver the active principle.

The main target of LH-RH peptide agonists according to formula (I) is the pituitary gland, but direct actions have been reported on the gonads themselves (testis and ovary), on the thymus and some lymphoid cell lines, and on breast, prostate, pancreatic or nervous system tumors. They exert on any LH-RH sensitive target, either a stimulatory activity by short-term acute or pulsatile administration, or an inhibitory effect by repeated or continuous administrations that induce the desensitization and the down-regulation of LH-RH receptors. In the case of the hypothalamo-pituitary-gonadal axis, prolonged administration results in a so-called 〈〈 chemical 〉〉 castration.

Accordingly, the oral pharmaceutical compositions of the invention can find an appropriate therapeutic use in humans as well as in animals, depending on doses and treatment regimens, in reproductive endocrinology and in the treatment or prevention of sex hormone-dependent benign or malignant tumors ; said treatment or prevention may involve parallel and/or sequential supplementary curative or preventive regimens based on other hormonal or antitumoral agents. LH-RH sensitive sex hormone-independent benign or malignant tumors can also regress upon treatment with the oral pharmaceutical compositions according to the invention, alone or associated with other parallel and/or sequential antitumoral treatments. Immune mechanisms can also be modified by the oral pharmaceutical compositions according to the invention, alone or associated with other parallel and/or sequential treatments based on immuno-modulating or -suppresive agents such as glucocorticoids, cyclosporin, rapamycin, tacrolimus, their derivatives, and the like. The oral pharmaceutical compositions according to the invention are therefore very valuable in the treatment and prevention of autoimmune diseases, graft rejection or atopic diseases, and in the treatment of benign or malignant lymphoproliferative disorders.

The oral pharmaceutical compositions according to the invention are especially useful in the inhibition, planning and triggering of ovulation in *in vitro* fertilization programs, and in the treatment of male and female infertility or hypogonadic states. Conversely, they can also be used in male or female contraception or treatment of hypergonadic states. In both cases, said treatments may involve other parallel and/or sequential treatments with sex steroids and/or gonadotrophins. This applies to men and women, but also to wild or domestic animals in uses such as improvement or control of reproductive performance, or as a tool to optimize breeding strategies.

The oral pharmaceutical compositions according to the invention are also especially useful in men to treat advanced prostate cancer, but can also be used as a first line therapy in this indication and in benign prostatic hypertrophy in both cases, said treatments may also involve additional parallel and/or sequential treatments based on inhibitors of androgen action, i.e. antiandrogens such as cyproterone acetate, osaterone acetate, chlormadinone acetate, flutamide, nilutamide or bicalutamide and the like, and/or on 5α-reductase inhibitors such as finasteride, epristeride or turosteride and the like, and/or on C₁₇₋₂₀ lyase inhibitors such as abiraterone and the like.

The oral pharmaceutical compositions according to the invention are also especially useful in the treatment or prevention of breast cancer in women and in men, especially estrogen receptor positive tumors ; said treatment or prevention may involve parallel or sequential supplementary curative or preventive regimens based on antiestrogens such as tamoxifen, raloxifen or droloxifen and the like, and/or on aromatase inhibitors such as atamestane, formestane, letrozole, anastrozole and the like, and/or on C₁₇₋₂₀ lyase inhibitors such as abiraterone and the like. The oral pharmaceutical compositions according to the invention are also very useful in the treatment or prevention of certain estrogen receptor negative tumors that respond to the direct effects of LH-RH analogues or indirectly to their gonadal suppressive activity.

Other gynecological conditions, such as endometrial hyperplasia, leiomyoma, adenomyoma, endometriosis, polycystic ovary syndrome, hirsutism and benign breast disease (pain, cysts or fibrosis), can also be prevented by or benefit from treatment with the oral pharmaceutical compositions according to the invention ; said treatment or prevention may involve additional parallel and/or sequential curative or preventive treatments based on antiestrogens (cited above), progestins such as cyproterone acetate, osaterone acetate, chlormadinone acetate, nomegestrol acetate, promegestone, demegestone, trimegestone and the like, and/or their contraceptive or post-menopausal replacement combination formulations with estrogens such as estradiol or ethynylestradiol. The oral compositions of the invention can also interfere with gestation by inducing abortion or by triggering labor ; in this case they may also be used in parallel or in sequence with treatments based on estrogens (cited above), antiprogestins such as mifepristone and/or prostaglandin analogs such as sulprostone.

Similar indications can be encountered in veterinary medicine for male or female domestic or wild animals that may require the use of pharmaceutical compositions according to the invention.

A further aspect of the invention relates to a method of treating and/or preventing the above diseases which comprises orally administering to patients or animals in need thereof a pharmaceutical composition according to the invention, said composition containing an effective amount of a LH-RH peptide analogue as previously defined. Said method may comprise the further administration of at least one of the active principles mentioned above such as for example a hormonal agent, an antitumoral agent, an immuno-modulating or -suppressive agent, a sex steroid, a gonadotrophin, an inhibitor of androgen action, a 5α-reductase inhibitor, a C₁₇₋₂₀ lyase inhibitor, an antiestrogen, an aromatase inhibitor, a progestin, an estrogen, an antiprogestin or a prostaglandin analogue, said further administration being parallel, sequential or over a period of time.

The unit dose of oral administration of LH-RH peptide agonists according to formula (I) may range from 0.1 to 100 mg per human patient, from one to 16 times per day (in the case of pulsatile administration), in combination with at least an equimolar amount of α-CD or its derivatives and up to the total remaining part of the oral formulation.

All the above-mentioned oral pharmaceutical compositions may additionally contain one or several proteases inhibitors, and/or one or several other absorption enhancers.

### Examples of preparations of leuprorelin, triptorelin, goserelin, deslorelin and examples 1 to 6 in combination with 100 mM α-CD in solution

On each experimental day, solutions of α-CD were freshly prepared by dissolving 9.72 g in 100 ml of pure water, or 4.86 g in 50 ml, for 1 hour at room temperature with gentle magnetic stirring; meanwhile, an appropriate volume of each LH-RH analogue was taken from thawed individual stock vials containing 50 µg of net peptide in 50 µl of phosphate-buffered saline containing 0.1 % bovine serum albumin, to make 20 nmoles (24.2 µl for LEU, 26.2 µl for TRI, 23.4 µl for GOS, 25.4 µl for DES, 24.7 µl for example 1, 23.6 µl for example 2, 24.5 µl for example 3, 25.1 µl for example 4, 26.5 µl for example 5 and 23.4 µl for example 6) and put in a 10 ml gauged flask. Then, the α-CD solution was added to fill the flask up to 10 ml to make a 2 nmol/ml solution of which 1ml was administered by oral gavage to each rat.

### Examples of preparation of formulations of example 2 in combination with 5%, 10% or 14% α-CD solutions

On each experimental day, 45 µl of one thawed individual vial containing 50 µg of net example 2 in 50 µl of phosphate-buffered saline containing 0.1 % bovine serum albumin, were diluted in 36 ml of distilled water to give a 1.25 µg/ml solution from which three fractions of 3.8 ml were taken; then, 190, 380 or 532 mg of α-CD were added to each fraction to give a concentration of 5%, 10% or 14%, respectively. After overnight magnetic stirring at room temperature, each solution was given to rats by oral gavage in a 4 ml/kg volume to administer the same dose of 5 µg/kg of example 2 without or with increasing concentrations of α-CD.

## Claims

1. Use of α-cyclodextrin or a derivative thereof for the preparation of a pharmaceutical composition for the oral administration of a LH-RH peptide analogue or one of its pharmaceutically acceptable salt.

2. Use according to claim 1 wherein said peptide analogue has the formula (SEQ ID N°: 1) :
A1-A2-A3-A4-A5-A6-A7-A8-Pro - Z (A)
in which :
- A1 is pGlu ; D-pGlu ; Sar ; AcSar ; Pro or a derivative thereof such as AcPro, ForPro, OH-Pro, Ac-OH-Pro, dehydro-Pro or Ac-dehydro-Pro ; Ser ; D-Ser ; Ac-D-Ser ; Thr ; D-Thr ; Ac-D-Thr ; or an aromatic D-amino acid which may be acylated ;
- A2 is a direct bond ; His ; or an aromatic D-amino acid ;
- A3 is an aromatic L- or D-amino acid ;
- A4 is Ala, Ser, D-Ser, MeSer, Ser(OBu^{t}), Ser(OBzl) or Thr;
- A5 is an aromatic L-amino acid ; or a basic L- or D-amino acid ;
- A6 is Gly ; (S)-spirolactam-Pro ; D-Pro ; D-Ser ; D-Thr ; D-Cys ; D-Met ; D-Pen ; D-(S-Me)Pen ; D-(S-Et)Pen ; D-Ser(OBu^{t}) ; D-Asp(OBu^{t}) ; D-Glu(OBu^{t}) ; D-Thr(OBu^{t}) ; D-Cys(OBu^{t}) ; D-Ser(OR₁) where R₁ is a sugar moiety ; an aza-amino acid such as azaGly or azaAla ; D-His which may be substituted on the imidazole ring by a (C₁-C₆)alkyl, a (C₂-C₇)acyl or a benzyl group ; an aliphatic D-amino acid with a (C₁-C₈)alkyl or a (C₃-C₆)cycloalkyl side chain ; an aromatic D-amino acid ; D-cyclohexadienyl-Gly ; D-perhydronaphthyl-Ala ; D-perhydrodiphenyl-Ala ; or a basic L- or D-amino acid ;
- A7 is a linear, branched or cyclic aliphatic L-amino acid of 3 to 20 carbon atoms which may be N-alpha-substituted by a (C₁-C₄)alkyl group optionally substituted by one or several fluorine atoms ;
- A8 is a basic L- or D-amino acid ;
- Z is GlyNH₂ ; D-AlaNH₂ ; azaGlyNH₂ ; or a group -NHR₂ where R₂ is a (C₁-C₄)alkyl which may be substituted by an hydroxy or one or several fluorine atoms ; a (C₃-C₆)cycloalkyl ; or a heterocyclic radical selected from morpholinyl, pyrrolidinyl and piperidyl.

3. Use according to claim 1 or 2 wherein said peptide analogue has the formula (SEQ ID N° : 2) :
A1-His-A3-A4-A5-A6-A7-A8-Pro-Z (I)
in which:
- A1 is pGlu, Sar or AcSar;
- A3 is an aromatic L-amino acid ;
- A4 is Ala, Ser, D-Ser, MeSer, Ser(OBu^{t}), Ser(OBzl) or Thr;
- A5 is an aromatic L-amino acid ;
- A6 is Gly; D-Pro ; (S)-spirolactam-Pro ; D-Ser ; D-Thr ; D-Cys ; D-Met ; D-Pen ; D-(S-Me)Pen ; D-(S-Et)Pen ; D-Ser(OBu^{t}) ; D-Asp(OBu^{t}) ; D-Glu(OBu^{t}) ; D-Thr(OBu^{t}) ; D-Cys(OBu^{t}) ; D-Ser(OR₁) where R₁ is a sugar moiety ; an aza-amino acid such as azaGly or azaAla ; D-His which may be substituted on the imidazole ring by a (C₁-C₆)alkyl or a benzyl group ; an aliphatic D-amino acid with a (C₁-C₈)alkyl or a (C₃-C₆)cycloalkyl side chain ; an aromatic D-amino acid ; D-cyclohexadienyl-Gly ; D-perhydronaphtyl-Ala ; D-perhydrodiphenyl-Ala ; or a basic D-amino acid ;
- A7 is a linear, branched or cyclic aliphatic L-amino acid of 3 to 20 carbon atoms which may be N-alpha-substituted by a (C₁-C₄)alkyl group optionally substituted by one or several fluorine atoms;
- A8 is a basic L-amino acid ;
- Z is GlyNH₂ ; azaGlyNH₂ ; or a group -NHR₂ where R₂ is a (C₁-C₄)alkyl which may be substituted by an hydroxy or one or several fluorine atoms ; a (C₃-C₆)cycloalkyl ; or a heterocyclic radical selected from morpholinyl, pyrrolidinyl and piperidyl.

4. Use according to claim 3 wherein said peptide analogue has the formula (SEQ ID N° : 3) :
pGlu-His-A3-Ser-A5-AG-A7-Arg-Pro-Z (II)
in which:
- A3 and A5 are aromatic L-amino acids ;
- A6 is as defined for (I) in claim 3 ;
- A7 is Leu, Tle, Nle, Hol, Npg, Cha or Ada, which may be N-alpha-substituted by a methyl or ethyl group optionally substituted by one or several fluorine atoms;
- Z is as defined for (I) in claim 3.

5. Use according to claim 4 wherein said peptide analogue has the formula (SEQ ID N° : 4) :
pGlu-His-A3-Ser-A5-A6-A7-Arg-Pro-Z (III)
in which:
- A3 and A5 are each independently Phe, Tyr, Trp, 2MeTrp, HPhe, HTyr, Nal, 1Nal, Bal, Pal, 4Pal, or pClPhe ;
- A6 is (S)-spirolactam-Pro ; Gly; D-Pro ; D-Ser(OBu^{t}) ; D-Asp(OBu^{t}) ; D-Glu(OBu^{t}) ; D-Thr(OBu^{t}) ; D-Cys(OBu^{t}); D-His or D-His(Bzl) ; D-Ala, D-Leu, D-Tle, D-Nle, D-Hol, D-Npg or D-Cha ; D-Phe, D-HPhe, D-Tyr, D-HTyr, D-Trp, D-2MeTrp, D-Nal, D-1Nal, D-Bal, D-Pal, D-4Pal, or D-pClPhe; D-cyclohexadienyl-Gly ; D-perhydronaphtyl-Ala ; D-perhydrodiphenyl-Ala ; or D-APhe optionally substituted by an aminotriazolyl group ;
- A7 is Leu, Npg or Cha, which may be N-alpha-substituted by a methyl group;
- Z is GlyNH₂ ; azaGlyNH₂ or -NC₂H₅.

6. Use according to one of claims 1 to 5 wherein the pharmaceutical composition is intended for the treatment of infertility, hypogonadic or hypergonadic states.

7. Use according to one of claims 1 to 5 wherein the pharmaceutical composition is a contraceptive agent.

8. Use according to one of claims 1 to 5 wherein the pharmaceutical composition is intended for the treatment or prevention of prostate cancer or benign prostatic hypertrophy.

9. Use according to one of claims 1 to 5 wherein the pharmaceutical composition is intended for the treatment or prevention of breast cancer.

10. Use according to one of claims 1 to 5 wherein the pharmaceutical composition is intended for the treatment or prevention of sex hormone-related benign or malignant tumors.

11. Use according to one of claims 1 to 5 wherein the pharmaceutical composition is intended for the treatment or prevention of sex hormone-independent but LH-RH sensitive benign or malignant tumors.

12. Use according to one of claims 1 to 5 wherein the pharmaceutical composition is intended for the treatment or prevention of benign or malignant lymphoproliferative disorders.

13. An oral pharmaceutical composition which comprises a therapeutically effective amount of a LH-RH peptide analogue in combination with α-cyclodextrin or a derivative thereof.

14. An oral pharmaceutical composition according to claim 13 wherein said peptide analogue has the formula (SEQ ID N° : 1) :
A1-A2-A3-A4-A5-A6-A7-A8-Pro - Z (A)
in which :
- A1 is pGlu ; D-pGlu ; Sar ; AcSar ; Pro or a derivative thereof such as AcPro, ForPro, OH-Pro, Ac-OH-Pro, dehydro-Pro or Ac-dehydro-Pro ; Ser ; D-Ser ; Ac-D-Ser ; Thr ; D-Thr ; Ac-D-Thr ; or an aromatic D-amino acid which may be acylated ;
- A2 is a direct bond ; His ; or an aromatic D-amino acid ;
- A3 is an aromatic L- or D-amino acid ;
- A4 is Ala, Ser, D-Ser, MeSer, Ser(OBu^{t}), Ser(OBzl) or Thr;
- A5 is an aromatic L-amino acid ; or a basic L- or D-amino acid ;
- A6 is Gly ; (S)-spirolactam-Pro ; D-Pro ; D-Ser; D-Thr; D-Cys; D-Met ; D-Pen ; D-(S-Me)Pen ; D-(S-Et)Pen ; D-Ser(OBu^{t}) ; D-Asp(OBu^{t}) ; D-Glu(OBu^{t}) ; D-Thr(OBu^{t}) ; D-Cys(OBu^{t}) ; D-Ser(OR₁) where R₁ is a sugar moiety ; an aza-amino acid such as azaGly or azaAla ; D-His which may be substituted on the imidazole ring by a (C₁-C₆)alkyl, a (C₂-C₄)acyl or a benzyl group ; an aliphatic D-amino acid with a (C₁-C₈)alkyl or a (C₃-C₆)cycloalkyl side chain ; an aromatic D-amino acid ; D-cyclohexadienyl-Gly ; D-perhydronaphthyl-Ala ; D-perhydrodiphenyl-Ala ; or a basic L- or D-amino acid ;
- A7 is a linear, branched or cyclic aliphatic L-amino acid of 3 to 20 carbon atoms which may be N-alpha-substituted by a (C₁-C₄)alkyl group optionally substituted by one or several fluorine atoms ;
- A8 is a basic L- or D-amino acid ;
- Z is GlyNH₂ ; D-AlaNH₂ ; azaGlyNH₂ ; or a group -NHR₂ where R₂ is a (C₁-C₄)alkyl which may be substituted by an hydroxy or one or several fluorine atoms ; a (C₃-C₆)cycloalkyl ; or a heterocyclic radical selected from morpholinyl, pyrrolidinyl and piperidyl.

15. An oral pharmaceutical composition according to claim 13 or 14 wherein said peptide analogue has the formula (SEQ ID N° : 2) :
A1-His-A3-A4-A5-A6-A7-A8-Pro-Z (I)
in which:
- A1 is pGlu, Sar or AcSar;
- A3 is an aromatic L-amino acid ;
- A4 is Ala, Ser, D-Ser, MeSer, Ser(OBu^{t}), Ser(OBzl) or Thr;
- A5 is an aromatic L-amino acid ;
- A6 is Gly; D-Pro; (S)-spirolactam-Pro ; D-Ser; D-Thr ; D-Cys ; D-Met ; D-Pen ; D-(S-Me)Pen ; D-(S-Et)Pen ; D-Ser(OBu^{t}) ; D-Asp(OBu^{t}) ; D-Glu(OBu^{t}) ; D-Thr(OBu^{t}) ; D-Cys(OBu^{t}) ; D-Ser(OR₁) where R₁ is a sugar moiety ; an an-amino acid such as azaGly or azaAla ; D-His which may be substituted on the imidazole ring by a (C₁-C₆)alkyl or a benzyl group ; an aliphatic D-amino acid with a (C₁-C₈)alkyl or a (C₃-C₆)cycloalkyl side chain ; an aromatic D-amino acid ; D-cyclohexadienyl-Gly ; D-perhydronaphtyl-Ala ; D-perhydrodiphenyl-Ala ; or a basic D-amino acid ;
- A7 is a linear, branched or cyclic aliphatic L-amino acid of 3 to 20 carbon atoms which may be N-alpha-substituted by a (C₁-C₄)alkyl group optionally substituted by one or several fluorine atoms;
- A8 is a basic L-amino acid ;
- Z is GlyNH₂ ; azaGlyNH₂ ; or a group -NHR₂ where R₂ is a (C₁-C₄)alkyl which may be substituted by an hydroxy or one or several fluorine atoms ; a (C₃-C₆)cycloalkyl ; or a heterocyclic radical selected from morpholinyl, pyrrolidinyl and piperidyl.

16. An oral pharmaceutical composition according to claim 15 wherein said peptide analogue has the formula (SEQ ID N° : 3) :
pGlu-His-A3-Ser-A5-A6-A7-Arg-Pro-Z (II)
in which:
- A3 and A5 are aromatic L-amino acids ;
- A6 is as defined for (I) in claim 15 ;
- A7 is Leu, Tle, Nle, Hol, Npg, Cha or Ada, which may be N-alpha-substituted by a methyl or ethyl group optionally substituted by one or several fluorine atoms;
- Z is as defined for (I) in claim 15.

17. An oral pharmaceutical composition according to claim 16 wherein said peptide analogue has the formula (SEQ ID N° : 4) :
pGlu-His-A3-Ser-A5-A6-A7-Arg-Pro-Z (III)
in which:
- A3 and A5 are each independently Phe, Tyr, Trp, 2MeTrp, HPhe, HTyr, Nal, 1Nal, Bal, Pal, 4Pal, or pClPhe ;
- A6 is (S)-spirolactam-Pro ; Gly; D-Pro ; D-Ser(OBu^{t}) ; D-Asp(OBu^{t}) ; D-Glu(OBu^{t}) ; D-Thr(OBu^{t}) ; D-Cys(OBu^{t}); D-His or D-His(Bzl) ; D-Ala, D-Leu, D-Tle, D-Nle, D-Hol, D-Npg or D-Cha; D-Phe, D-HPhe, D-Tyr, D-HTyr, D-Trp, D-2MeTrp, D-Nal, D-1Nal, D-Bal, D-Pal, D-4Pal, or D-pClPhe; D-cyclohexadienyl-Gly ; D-perhydronaphtyl-Ala ; D-perhydrodiphenyl-Ala or D-APhe optionally substituted by an aminotriazolyl group ;
- A7 is Leu, Npg or Cha, which may be N-alpha-substituted by a methyl group;
- Z is GlyNH₂ ; azaGlyNH₂ or -NC₂H₅.

18. An oral pharmaceutical composition according to one of claims 13 to 17 which further comprises a protease inhibitor and/or an absorption enhancer.


SEQUENCE LISTING

(1) GENERAL INFORMATION:

(i) APPLICANT:
(A) NAME: Laboratoire Theramex
(B) STREET: 6, Avenue du Prince Hereditaire Albert
(C) CITY: Monaco
(E) COUNTRY: Monaco
(F) POSTAL CODE (ZIP): 98000
(G) TELEPHONE: 377 92 05 08 08
(H) TELEFAX: 377 92 05 70 00

(ii) TITLE OF INVENTION: Pharmaceutical compositions based on α-
cyclodextrin for the oral administration of LH-RH analogues.

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 10 amino acids
(B) TYPE: amino acid
(D) TOPOLOGY: linear

(ii) MOLECULE TYPE: peptide

(ix) FEATURE:
(A) NAME/KEY: Modified-site
(B) LOCATION:1
(D) OTHER INFORMATION:/note= "Xaa is pGlu ; D-pGlu ; Sar
; AcSar ; Pro·; AcPro ; ForPro ; OH-Pro ; Ac-OH-Pro ; dehydro-Pro ;
Ac-dehydro-Pro ; Ser ; D- Ser ; Ac-D-Ser ; Thr ; D-Thr ; Ac-D-Thr ;
or an optionally acylated aromatic D-amino acid"

(ix) FEATURE:
(A) NAME/KEY: Modified-site
(B) LOCATION:2
(D) OTHER INFORMATION:/note= "Xaa is a direct bond ; His ;
or an aromatic D-amino acid"

(ix) FEATURE:
(A) NAME/KEY: Modified-site
(B) LOCATION:3
(D) OTHER INFORMATION:/note= "Xaa is an aromatic L- or D-
amino acid"

(ix) FEATURE:

(A) NAME/KEY: Modified-site
(B) LOCATION:4
(D) OTHER INFORMATION:/note= "Xaa is Ala, Ser, D-Ser,
MeSer, Ser(OBu^t), Ser(OBzl) or Thr"

(ix) FEATURE:
(A) NAME/KEY: Modified-site
(B) LOCATION:5
(D) OTHER INFORMATION:/note= "Xaa is an aromatic L-amino
acid or a basic L- or D-amino acid"

(ix) FEATURE:
(A) NAME/KEY: Modified-site
(B) LOCATION:6
(D) OTHER INFORMATION:/note= "Xaa is Gly ; (S)spirolactam-
Pro ; D-Pro ; D-Ser ; D-Thr ; D-Cys ; D-Met ; D-Pen ; D-(S-Me)Pen ;
D-(S-Et)Pen ; D-Ser(OBu^t) ; D-Asp(OBu^t) ; D-Glu(OBu^t) ; D-Thr(OBu^t) ;
D-Cys(OBu^t) ; D-Ser(OR_1) where R_1 is a sugar moiety ; an aza-amino
acid; D-His which may be substituted on the imidazole ring ; an
aliphatic D-amino acid with a (C_1-C_8)alkyl or a (C_3-C_6)cycloalkyl
side chain ; an aromatic D-amino acid ; D-cyclohexadienyl-Gly ; D-
perhydronaphthyl-Ala ; D-perhydrodiphenyl-Ala ; or a basic L- or D-
amino acid"

(ix) FEATURE:
(A) NAME/KEY: Modified-site
(B) LOCATION:7
(D) OTHER INFORMATION:/note= "Xaa is a linear, branched or
cyclic aliphatic C_3-C_20 L-amino acid which may be N-alpha-
substituted"

(ix) FEATURE:
(A) NAME/KEY: Modified-site
(B) LOCATION:8
(D) OTHER INFORMATION:/note= "Xaa is a basic L- or D-amino
acid"

(ix) FEATURE:
(A) NAME/KEY: Modified-site
(B) LOCATION:10
(D) OTHER INFORMATION:/note= "Xaa is GlyNH_2 ; D-AlaNH_2 ;
azaGlyNH_2 ; or a group -NHR_2 where R_2 is an optionally substituted
(C_1-C_4)alkyl "

(xi) SEQUENCE DESCRIPTION: SEQ ID NO: 1:

Xaa Xaa Xaa Xaa Xaa Xaa Xaa Xaa Pro Xaa
1               5                   10

(2) INFORMATION FOR SEQ ID NO: 2:

(i) SEQUENCE CHARACTERISTICS:
(A) LENGTH: 10 amino acids
(B) TYPE: amino acid

(D) TOPOLOGY: linear

(ii) MOLECULE TYPE: peptide

(ix) FEATURE:
(A) NAME/KEY: Modified-site
(B) LOCATION:1
(D) OTHER INFORMATION:/note= "Xaa is pGlu, Sar or AcSar"

(ix) FEATURE:
(A) NAME/KEY: Modified-sites
(B) LOCATION:3,5
(D) OTHER INFORMATION:/note= "Xaa is an aromatic L-amino
acid"

(ix) FEATURE:
(A) NAME/KEY: Modified-sites
(B) LOCATION:4,7
(D) OTHER INFORMATION:/note= "Xaa is as defined for SEQ ID
NO:1"

(ix) FEATURE:
(A) NAME/KEY: Modified-site
(B) LOCATION:6
(D) OTHER INFORMATION:/note= "Xaa is Gly ; (S)spirolactam-
Pro ; D-Pro ; D-Ser ; D-Thr ; D-Cys ; D-Met ; D-Pen ; D-(S-Me)Pen ;
D-(S-Et)Pen ; D-Ser(OBu^t) ; D-Asp(OBu^t) ; D-Glu(O-Bu^t) ; D-Thr(O-Bu^t)
; D-Cys(O-Bu^t) ; D-Ser(O-R_1) where R_1 is a sugar moiety ; an aza-
amino acid ; D-His which may be substituted on the imidazole ring ;
an aliphatic D-amino acid with a (C_1-C_8)alkyl or a (C_3-C_6)cycloalkyl
side chain ; an aromatic D-amino acid ; D-cyclohexadienyl-Gly ; D-
perhydronaphthyl-Ala ; D-perhydrodiphenyl-Ala ; or a basic D-amino
acid"

(ix) FEATURE:
(A) NAME/KEY: Modified-site
(B) LOCATION:8
(D) OTHER INFORMATION:/note= "Xaa is a basic L-amino acid"

(ix) FEATURE:
(A) NAME/KEY: Modified-site
(B) LOCATION:10
(D) OTHER INFORMATION:/note= "Xaa is as defined for SEQ ID
NO:1"

(xi) SEQUENCE DESCRIPTION: SEQ ID NO: 2:

Xaa His Xaa Xaa Xaa Xaa Xaa Xaa Pro Xaa
1               5                   10

(2) INFORMATION FOR SEQ ID NO: 3:

(i) SEQUENCE CHARACTERISTICS:
(A) LENGTH: 10 amino acids

(B) TYPE: amino acid
(D) TOPOLOGY: linear

(ii) MOLECULE TYPE: peptide

(ix) FEATURE:
(A) NAME/KEY: Modified-site
(B) LOCATION:1
(D) OTHER INFORMATION:/note= "Xaa is pGlu"

(ix) FEATURE:
(A) NAME/KEY: Modified-sites
(B) LOCATION: 3,5,6,10
(D) OTHER INFORMATION:/note= "Xaa is as defined for SEQ ID
NO:2"

(ix) FEATURE:
(A) NAME/KEY: Modified-site
(B) LOCATION:7
(D) OTHER INFORMATION:/note= "Xaa is Leu, Tle, Nle, Hol,
Npg, Cha or Ada which may be N-alpha substituted"

(xi) SEQUENCE DESCRIPTION: SEQ ID NO: 3:

Xaa His Xaa Ser Xaa Xaa Xaa Arg Pro Xaa
1               5                   10

(2) INFORMATION FOR SEQ ID NO: 4:

(i) SEQUENCE CHARACTERISTICS:
(A) LENGTH: 10 amino acids
(B) TYPE: amino acid
(D) TOPOLOGY: linear

(ii) MOLECULE TYPE: peptide

(ix) FEATURE:
(A) .NAME/KEY: Modified-site
(B) LOCATION:1
(D) OTHER INFORMATION:/note= "Xaa is pGlu"

(ix) FEATURE:
(A) NAME/KEY: Modified-sites
(B) LOCATION:3,5
(D) OTHER INFORMATION:/note= "Xaa is Phe, Tyr, Trp,
2MeTrp, HPhe, HTyr, Nal, 1Nal, Bal, Pal, 4Pal or pClPhe"

(ix) FEATURE:
(A) NAME/KEY: Modified-site
(B) LOCATION: 6
(D) OTHER INFORMATION:/note= "Xaa is (S)-spirolactam-Pro ;
Gly; D-Pro ; D-Ser(OBu^t) ; D-Asp(OBu^t) ; D-Glu(OBu^t) ; D-Thr(OBu^t) ;
D-Cys(OBu^t); D-His or D-His(Bzl) ; D-Ala, D-Leu, D-Tle, D-Nle, D-
Hol, D-Npg or D-Cha ; D-Phe, D-HPhe, D-Tyr, D-HTyr, D-Trp, D-2MeTrp,

D-Nal, D-1Nal, D-Bal, D-Pal, D-4Pal, or D-pClPhe; D-cyclohexadienyl-
Gly ; D-perhydronaphtyl-Ala ; D-perhydrodiphenyl-Ala or D-APhe
optionally substituted"

(ix) FEATURE:
(A) NAME/KEY: Modified-site
(B) LOCATION:7
(D) OTHER INFORMATION:/note= "Xaa is Leu, Npg or Cha which
may be N-alpha-methylated"

(ix) FEATURE:
(A) NAME/KEY: Modified-site
(B) LOCATION:10
(D) OTHER INFORMATION:/note= "Xaa is GlyNH2, azaGlyNH2 or
-NHC2H5"

(xi) SEQUENCE DESCRIPTION: SEQ ID NO: 4:

Xaa His Xaa Ser Xaa Xaa Xaa Arg Pro Xaa
1               5                   10